(19) Europäisches Patentamt
European Patent Office
Office européen des brevets

(11) Numéro de publication: 0 208 623
A2

(12) DEMANDE DE BREVET EUROPEEN

(21) Numéro de dépôt: 86401562.3

(51) Int. Cl.[4]: A 61 K 31/725

(22) Date de dépôt: 11.07.86

(30) Priorité: 12.07.85 FR 8510788

(43) Date de publication de la demande:
14.01.87 Bulletin 87/3

(84) Etats contractants désignés:
AT BE CH DE FR GB IT LI LU NL SE

(71) Demandeur: SANOFI
40, Avenue George V
F-75008 Paris(FR)

(72) Inventeur: Robert, Ladislas
7, rue Jean-Baptiste Lulli
F-94440 Santeny(FR)

(72) Inventeur: Hornbeck, William
10, bld. du Roi
F-78000 Versailles(FR)

(72) Inventeur: Petitou, Maurice
65, rue du Javelot
F-75013 Paris(FR)

(72) Inventeur: Choay, Jean
21, rue Saint-Guillaume
F-75007 Paris(FR)

(74) Mandataire: Peaucelle, Chantal et al,
S.C. Ernest Gutmann - Yves Plasseraud 67, boulevard Haussmann
F-75008 Paris(FR)

(54) Utilisation de poly- et oligosaccharides pour l'obtention de médicaments actifs dans les pathologies du tissu conjonctif.

(57) Selon l'invention, on utilise un glycosaminoglycane ou un fragment de glycosaminoglycane, éventuellement sous forme de sel, pour élaborer un médicament actif vis-à-vis des pathologies du tissu conjonctif.

EP 0 208 623 A2

./...

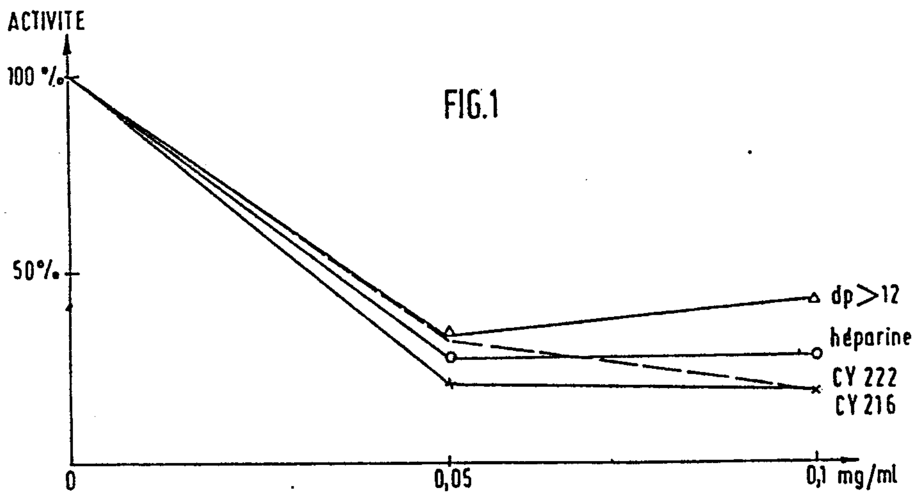


FIG.1

Utilisation de poly- et oligosaccharides pour l'obtention de médicaments actifs dans les pathologies du tissu conjonctif.

L'invention a pour objet l'utilisation de poly- et oligosaccharides pour l'obtention de médicaments actifs dans les pathologies du tissu conjonctif.

Parmi ces pathologies, les maladies cardio-vasculaires, ostéo-articulaires et pulmonaires et autres maladies du vieillissement et également les processus inflammatoires de façon générale, ainsi que de nombreuses tumeurs malignes, entraînent des incapacités dont la fréquence et la gravité augmentent souvent exponentiellement avec l'âge.

Les mécanismes exacts de ces maladies ne sont pas complètement élucidés. Il est cependant admis qu'il existe un certain nombre de mécanismes cellulaires et moléculaires connus impliquant, notamment des déséquilibres de rapports protéases/antiprotéases à activité élastinolytique, plus spécialement des rapports élastases (en particulier élastase leucocytaire)/anti-élastases.

On sait, par exemple, que certaines maladies pulmonaires, telles que l'emphysème, ou des maladies artérielles, telles que l'artériosclérose, impliquent des systèmes élastase/élastine et leurs inhibiteurs.

Il est généralement admis que l'élastolyse pulmonaire est une étape initiale et fondamentale dans le développement de l'emphysème. Cette élastolyse serait due à une diminution du taux des inhibiteurs d'élastases ou à leur inactivation, ou encore à une augmentation de l'activité élastolytique locale tissulaire.

Le rapport élastases/inhibiteurs d'élastases

constitue donc un facteur capital dans le développement de cette pathologie.

Les élastases le plus souvent impliquées à cet égard correspondent à l'élastase leucocytaire et à l'élastase des monocytes-macrophages. Il s'agit respectivement d'une sérine-protéase et d'une métallo-protéase.

Dans les autres maladies évoquées, on note également une augmentation de l'activité de certaines protéases, souvent de protéases de type élastase. Une augmentation exponentielle de la biosynthèse d'une protéase de type élastase (sérine-protéase) ancrée dans la membrane cellulaire des cellules musculaires lisses vasculaires, l'existence d'une élastase de type métallo-protéase dans les fibroblastes pouvant être excrétée dans le milieu et augmentant également avec le nombre de doublements de population cellulaire, c'est-à-dire avec le vieillissement in vitro, ont été démontrées récemment.

Le rapport de ces élastases et de leurs inhibiteurs est en déséquilibre soit parce que l'élastase est secrétée en quantité trop importante, soit parce qu'il existe un défaut en inhibiteurs d'élastase, et notamment une déficience en alpha-1 protéinase inhibiteur, ou alpha 1 anti-trypsine, désignée ci-après par alpha $_1$PI. Ce déséquilibre peut encore résulter de l'adsorption rapide de l'élastase sur les fibres élastiques et de son inaccessibilité aux inhibiteurs.

Jusqu'à présent, ces maladies n'ont pas trouvé de médication valable universellement acceptée et appliquée.

Pour traiter par exemple l'emphysème, on utilise généralement des inhibiteurs naturels ou de synthèse. Comme inhibiteur naturel, il s'agit principalement de l'alpha-1 PI provenant d'un donneur. Cet inhibiteur est

perfusé chez un receveur emphysémateux. Etant donné la faible période biologique de l'alpha 1 PI, il est nécessaire d'effectuer ce traitement de façon répétitive, ce qui le rend difficilement applicable sur le plan clinique.

Les inhibiteurs synthétiques des élastases efficaces in vitro, tels que les peptides chlorométhylcétones ou les fluorures de sulfonyle, présentent des inconvénients majeurs in vivo. Leur taux de sélectivité est en effet peu élevé, leur toxicité est loin d'être négligeable et, en outre, ils ne sont efficaces que s'ils sont injectés très peu de temps avant ou après l'injection intratrachéale d'élastase induisant l'emphysème (leur efficacité dépend, en effet, de la rapidité du contact avec le site actif de l'enzyme).

Les travaux effectués par les inventeurs dans ce domaine les ont amenés à constater qu'en utilisant certains poly- et oligosaccharides déjà connus pour leurs propriétés régulatrices vis-à-vis de la coagulation sanguine, il est possible d'obtenir des médicaments actifs sur les protéases à activité élastinolytique, de sélectivité élevée, de grande efficacité et pratiquement dépourvus d'effets secondaires.

L'invention a donc pour but de fournir de nouveaux médicaments utilisables dans les pathologies du tissu conjonctif impliquant des déséquilibres protéases anti-protéases. Elle vise également leur utilisation sous différentes formes d'administration pour prévenir et traiter les facteurs responsables des déséquilibres de ces systèmes.

Conformément à l'invention, on utilise, pour l'obtention d'un médicament actif vis-à-vis des pathologies du tissu conjonctif, un glycosaminoglycane ou un fragment de glycosaminoglycane, le cas échéant sous forme de sel pharmacologiquement acceptable.

On rappelle que les glycosaminoglycanes ou GAGs sont essentiellement formés de motifs alternés acide uronique-sucre aminé (ou l'inverse), du type de ceux rencontrés dans les chaînes oligo- et polysaccharidiques de GAGs naturels biologiquement actifs comme l'héparine, ou l'héparane-sulfate, les chondroïtines, les chondroïtines-sulfates ou l'acide hyaluronique. Dans ces chaînes, les motifs acide uronique répondent plus spécialement à la structure acide D-glucuronique ou L-iduronique et les motifs sucre aminé à celle de la D-glucosamine (dans l'héparine ou l'héparane-sulfate par exemple), ou de la D-galactosamine dans les autres produits cités.

Dans la description et les revendications, le terme GAG est utilisé aussi bien pour désigner les glycosaminoglycanes naturels, leurs fragments ou chaînes de bas poids moléculaires, ou leurs sels pharmacologiquement acceptables.

On sait que certains produits naturels renferment des chaînes possédant une affinité élevée pour l'antithrombine III (AT-III) où le co-facteur II de l'héparine (HCII).

Par extraction alcoolique ou par dépolymérisation nitreuse ou enzymatique, il est possible d'augmenter le taux de ces chaînes et d'élaborer des GAGs ayant une activité plus spécifique vis-à-vis du facteur Xa de la coagulation.

Ces compositions sont caractérisées par des rapports du titre Yin-Wessler (mesure de l'activité anti-Xa qui exprime l'activité antithrombotique) et du titre USP (mesure de l'activité anticoagulante globale) plus élevé que l'héparine, c'est-à-dire supérieur à 1.

Le titre USP peut devenir pratiquement nul chez certains GAGs et le titre YW dépasser des valeurs de 2000 u/mg.

D'une manière surprenante, l'étude in vitro et

in vivo de ces GAGs a montré leur efficacité sur différentes élastases purifiées.

Plus spécialement, ces produits exercent une forte inhibition, de grande sélectivité, de l'élastase leucocytaire, enzyme principalement impliquée dans les pathologies du tissu conjonctif liées à des déséquilibres du rapport élastase/anti-élastase.

D'une manière avantageuse, cette inhibition s'étend à la cathepsine G, qui se trouve avec l'élastase leucocytaire dans les leucocytes polymorphonucléaires et qui potentialise son activité au niveau des lésions tissulaires, inflammatoires aiguës ou chroniques.

Selon un autre aspect de grand intérêt, ces produits sont capables d'augmenter le taux sérique des inhibiteurs d'élastase.

Selon un mode de réalisation de l'invention, le GAG utilisé pour l'élaboration de médicaments selon l'invention est à base des motifs alternés D-glucosamine-acide uronique (acide L-iduronique ou D-glucuronique) tels que rencontrés dans l'héparine ou l'héparane-sulfate.

Dans un mode préféré de mise en oeuvre, il s'agit d'héparine.

Dans un autre mode de mise en oeuvre préféré, le GAG utilisé est constitué par de l'héparan sulfate.

Dans un autre mode préféré encore, le GAG de départ possède un poids moléculaire inférieur à celui de l'héparine ou de l'héparan sulfate.

En particulier, le poids moléculaire (PM) est inférieur à 10 000 daltons environ.

Ces GAGs de bas poids moléculaire ou bas PM comprennent, notamment, les produits suivants, à savoir :

- Les mucopolysaccharides tels qu'obtenus par extraction alcoolique, à partir d'héparine, selon le

brevet FR 2 440 376 du 6.11.1978 et le premier certificat d'addition 2 461 719 du 20.07.1979.

Ces produits, selon l'un des aspects envisagés dans le brevet principal, sont formés d'un mélange de chaînes de poids moléculaires d'environ 2000 à 8000 et présentent des rapports des titres Yin Wessler/USP d'au moins 2. Parmi les produits décrits, on citera ceux possédant un rapport YW/USP de l'ordre de 3 à 5 et des poids moléculaires moyens de 3000 à 5500 appelés ci-après CY 216.

- Les mucopolysaccharides tels qu'obtenus par dépolymérisation ménagée de l'héparine à l'aide d'acide nitreux, selon notamment le deuxième certificat d'addition 2 478 646 du 20.03.1980 au brevet FR 2 440 376 ci-dessus ou la demande de brevet FR 2 503 714 du 10.04.-1981. Ces produits répondent d'une manière générale aux caractéristiques ci-dessus mais sont terminés par des groupes de structure 2,5-anhydro-manno.

On citera notamment les mucopolysaccharides du type possédant une extrémité 2,5-anhydro-mannitol, appelés ci-après CY 222, ou encore une extrémité acide 2,5-anhydro-mannonique. Les poids moléculaires des chaînes glycosidiques majoritaires sont plus spécialement de l'ordre de 2000 à 3000, notamment d'environ 2000 à 2600. Les rapports des titres Yin-Wessler/USP sont notamment d'au moins 10 avec des titres Yin-Wessler d'au moins environ 200 u/mg.

- Les mucopolysaccharides essentiellement formés de chaînes (1) d'un poids moléculaire moyen de 3000 à 6000 daltons, notamment de 4000 à 5000 environ (2) possédant un titre YW/USP inférieur à 10, notamment d'environ 6 à 3, (3) terminées par des motifs de structure 2,5-anhydromanno, tels qu'obtenus par exemple selon la demande de brevet FR 2 572 080 du 18.10.1984.

- Les oligosaccharides formés de 8 motifs sucre

au plus, de titre Yin-Wessler élevé pouvant atteindre 2000 u/mg et de titre USP faible, pratiquement nul, dotés d'une forte affinité pour l'AT-III, tels que décrits notamment dans la demande EP 0 027 089 du 6.10.1980.

Selon une variante, ces oligosaccharides sont obtenus par dépolymérisation nitreuse ménagée et sont terminées par des groupes de structure 2,5-anhydromanno.

Selon une autre variante, le motif de début de chaîne correspond à un motif uronique insaturé, tel que formé en soumettant l'héparine à une dépolymérisation ménagée à l'aide d'une héparinase. En particulier, ces oligosaccharides sont formés des octosaccharides de structure ABCDEFGH répondant à la formule ci-dessous :

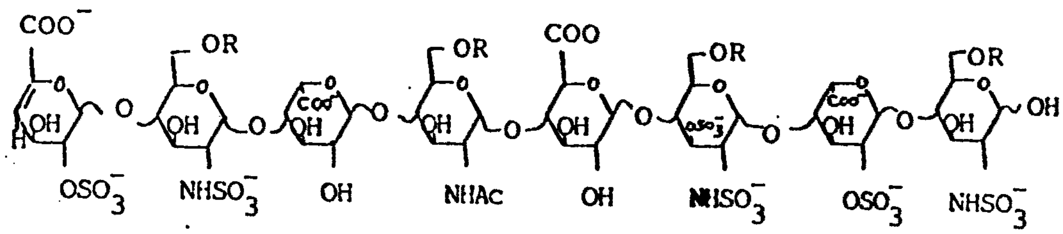

A B C D E F G H

- Des compositions homogènes d'hexasaccharides, telles qu'obtenues selon la demande FR 2 504 928 du 29.04.1981 en soumettant les octasaccharides ci-dessus à l'action ménagée d'une héparinase. En particulier, ces compositions répondent à la formule ci-dessous :

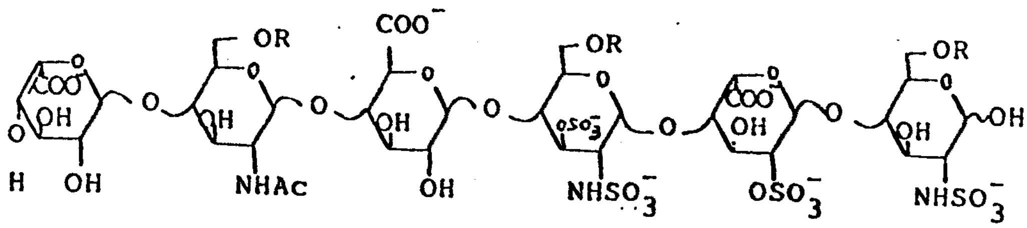

C' D E F G H

- Des oligo- ou polysaccharides de degré de polymérisation homogène, notamment ceux obtenus par filtration, sur un matériau tel que du Séphadex, ou par chromatographie par gradient de force ionique, des produits CY 222, CY 216 héparine, ou d'autres GAGs.

- Des GAGs tels qu'obtenus par action de periodate sur l'héparine, suivie de traitement en milieu basique.

- Des GAGs dits totaux, correspondant aux compositions de GAGs obtenues par traitement d'organes animaux.

- Des GAGs tels qu'obtenus par dépolymérisation de l'héparine avec de l'héparinase ou de l'acide nitreux, séparation des fractions possédant la séquence de fixation à l'AT-III et gel filtration de ces fractions afin d'obtenir des fragments de GAGs de degré de polymérisation varié.

Il peut s'agir, par exemple, de fragments de dp supérieur à 12, d'une part, et d'autre part de fragments de GAGs de degré de polymérisation inférieur, notamment de dp 12, 10, 8, 6 et 4.

Ces différents GAGs ne sont cités qu'à titre d'exemples, étant entendu qu'ils correspondent à des produits particulièrement bien connus de la Demanderesse qui les a mis au point et décrits dans les différents brevets et demandes de brevets ci-dessus déposés à son nom.

Tout autre glycosaminoglycane peut être mis en oeuvre pour l'élaboration des médicaments de l'invention.

Ainsi, selon encore un autre mode de réalisation de l'invention, le GAG mis en oeuvre est à base de motifs alternés D-galactosamine-acide uronique, tels que rencontrés par exemple dans le dermanatane sulfate, les chondroïtines, les chondroïtines-sulfates ou encore

l'acide hyaluronique.

Dans une variante, il s'agit avantageusement de dermatane-sulfate.

Selon une autre variante, le GAG mis en oeuvre est formé de chondroïtines ou de chondroïtines-sulfates.

Dans une autre variante encore, le GAG est constitué par de l'acide hyaluronique.

Selon une autre disposition, les GAGs de départ sont homogènes au regard de leur degré de polymérisation. Ces GAGs sont obtenus, par exemple, par filtration sur un gel d'un mélange de dépolymérisation d'héparine. Cette séparation est facilitée par l'utilisation de mélanges de dépolymérisation formés de chaînes de degré de polymérisation pair.

Selon une variante, dont la mise en oeuvre est également facilitée par l'utilisation de mélanges de dépolymérisation formés de chaînes de degré de polymérisation pair, on soumet le mélange de GAGs à une opération de gel filtration afin d'obtenir des GAGs homogènes au regard de leur degré de polymérisation.

Selon encore un autre mode de réalisation de l'invention, on utilise un GAG dont le profil de sulfatation est modifié.

Il s'agit de produits tels que définis ci-dessus dans lesquels une partie, voire la majorité et même la totalité des groupes -OH sont remplacés par des groupes esters sulfuriques $SO_3^-$.

Des produits de ce type sont notamment décrits dans la demande FR 8 510 787 du 12.07.85 déposée ce jour au nom de la demanderesse et sont avantageusement obtenus, selon le procédé décrit dans cette demande de brevet, par sulfatation d'un GAG donné, mis en oeuvre sous forme d'un sel soluble en milieu organique.

Conformément à l'aspect le plus général de ce procédé, on transforme un GAG donné en un sel soluble

dans un solvant organique, on le solubilise dans un solvant organique et on traite le sel en solution par un agent de sulfatation.

Le degré de sulfatation varie d'environ 2 jusqu'à des valeurs d'environ 4.

Ces GAGs comprennent les produits dans lesquels une partie ou encore la majorité ou même la totalité des groupes $-NHSO_3^-$ en position 2 des motifs sucre aminé est remplacée par des groupes -NH-acyle, notamment -NH-acétyle.

L'étude de l'action des GAGs ci-dessus dans des réactions modèles in vitro ou dans des modèles animaux in vivo a mis en évidence leur grande efficacité.

Les travaux effectués à cet égard ont permis de mettre en évidence une forte inhibition, hautement sélective, et à faible concentration (0,5 ng/ml) de l'élastase leucocytaire ainsi que de la cathepsine G (capable de potentialiser l'activité de l'élastase). Une autre constatation importante concerne la bonne corrélation entre l'inhibition exercée par ces produits sur l'élastase leucocytaire et la cathepsine G.

Les essais in vivo effectués sur le hamster, décrits dans les exemples qui suivent, ont montré qu'ils inhibent fortement l'activité élastasique du sérum et selon un aspect de grande importance qu'ils conduisent à une augmentation progressive du taux sérique des inhibiteurs d'élastase, en particulier de l'$alpha_1$PI.

Le spectre d'efficacité de ces GAGs s'étend à l'inhibition de la biosynthèse de la fibronectine qui constitue un facteur d'adhésion celulaire intervenant dans l'athérogénèse.

Ces GAGs agissent également sur l'inhibition de la synthèse du collagène, en particulier du collagène de type I + III.

Une autre propriété des GAGs est d'interférer

avec la précipitation des lipoprotéines légères (LDL) par des protéoglycanes artériels. Il est généralement admis que la formation de complexes insolubles libres LDL-protéoglycanes artériels intervient après la formation des dépôts athéromateux. Les phénomènes énumérés ci-dessus sont considérés dans leur ensemble comme importants dans le développement de l'artériosclérose et l'obstruction des vaisseaux.

L'intérêt de ces propriétés s'ajoute à la non toxicité connue de ces produits, ce qui les rend particulièrement précieux pour l'élaboration de médicaments utilisables dans des pathologies du tissu conjonctif, en particulier en pathologie pulmonaire, vasculaire et articulaire.

Les préparations pharmaceutiques selon l'invention sont caractérisées en ce qu'elles renferment une quantité efficace des GAGs définis ci-dessus en association avec des excipients pharmaceutiques. Ces préparations pharmaceutiques sont dépourvues de substances pyrogènes.

Des compositions préférées comprennent un véhicule pharmaceutique approprié pour l'administration par voie orale. Des formes d'administration de l'invention correspondantes comprennent avantageusement des gélules gastrorésistantes, des comprimés ou tablettes, des pilules, ou encore se présentent sous forme de liposomes.

D'autres compositions pharmaceutiques comprennent ces GAGs en association avec les excipients appropriés pour l'administration par voie rectale. Des formes d'administration correspondantes sont constituées par des suppositoires.

Dans d'autres compositions pharmaceutiques, les GAGs se présentent sous forme d'aérosols ou de pommades.

L'invention concerne également des compositions pharmaceutiques injectables, stériles ou stérilisables

pour l'administration tant par voie intraveineuse qu'intramusculaire ou sous-cutanée.

Ces solutions renferment avantageusement de 1 à 200 mg/ml de GAGs, de préférence de 20 à 150 mg/ml lorsque ces solutions sont destinées à l'injection par voie sous-cutanée. Elles peuvent contenir, par exemple, de notamment 30 à 100 mg/ml, notamment de 40 à 50 mg/ml de GAGs lorsqu'elles sont destinées à l'injection par voie intraveineuse ou par perfusion.

Avantageusement, de telles préparations pharmaceutiques sont présentées sous la forme de seringues non récupérables, prêtes à l'emploi.

Les compositions pharmaceutiques de l'invention sont particulièrement adaptées pour prévenir ou traiter les maladies cardio-vasculaires, ostéo-articulaires, pulmonaires ou autres maladies du vieillissement impliquant des lésions du tissu conjonctif.

Ces compositions sont plus spécialement appropriées pour prévenir ou traiter les déséquilibres élastase, en particulier élastase leucocytaire-anti-élastase.

Elles revêtent également un grand intérêt dans l'athérogénèse étant donné également leur effet inhibiteur vis-à-vis de la fibronectine, du collagène ainsi que de l'interaction avec les LDL.

Elles permettent en outre de réduire le taux de collagène dans les tissus dont la quantité augmente, par exemple, dans les artérioscléroses, en particulier dans les parois vasculaires, dans les fibroses pulmonaires (dans les poumons) et dans les cirrhoses (dans le foie).

Leur activité antiélastasique les rend également appropriés pour traiter le développement des maladies vasculaires, dans lesquelles l'attaque des lames basales des capillaires est à la base des accidents vasculaires et cérébraux, ou encore des maladies ostéoarticulaires.

Afin d'illustrer l'invention, on indique un exemple de posologie utilisable chez l'homme : cette posologie comprend l'administration d'environ 1 mg à 1,5 g/24 heures selon la voie d'administration, de préférence de 5 à 500 mg/jour environ, par exemple de l'ordre de 200 mg/24 heures par voie intraveineuse ou encore 100 à 500 mg/jour par voie orale.

Ces doses peuvent être naturellement ajustées pour chaque patient en fonction des résultats et des analyses de sang effectuées auparavant, la nature des affections dont il souffre et, d'une manière générale, de son état de santé.

L'invention concerne également encore l'application des GAGs selon l'invention à la constitution de réactifs biologiques utilisables au laboratoire, notamment à titre de référence de comparaison pour l'étude d'autres produits dont est testée l'activité inhibitrice de protéases.

D'autres caractéristiques et avantages de l'invention apparaîtront dans les exemples qui suivent.

<u>EXEMPLE 1</u> : Etude in vitro sur l'activité élastolytique et la capacité d'inhibition des anti-protéases sériques.

a) Dosage de l'activité élastolytique sérique :

Le principe du dosage est le suivant : après rupture protéolytique de la liaison amidique entre l'acide aminé et le composé paranitroanilide, ce dernier est libéré.

L'activité élastasique est alors estimée colorimétriquement à 410 nm, et est proportionnelle à la quantité de moles de substrat hydrolysé.

On prépare une solution 125 mM de substrat synthétique, le succinoyl-trialanyl-paranitroanilide ou STANA, dans la N-méthyl-2-pyrrolidone, à raison de 79 mg pour 1 ml.

On ajoute des quantités croissantes d'élastase

et de tampon (Tris/HCl 0,1M pH 8, Na $N_3$ 0,02 % détergent de Brij 0,1 %, NaCl0,1 M).

On incube les préparations pendant une heure à 37°C, puis on arrête la réaction en ajoutant 20 µl de PMSF.

On détermine l'activité enzymatique en mesurant la densité optique en fonction de la concentration en élastase (les quantités d'enzymes sont de l'ordre du nanogramme).

b) Dosage de la capacité d'inhibition sérique vis-à-vis de l'élastase.

- Principe du dosage.

On titre l'activité élastosique résiduelle après avoir ajouté des quantités d'inhibiteurs croissantes contenus dans le sérum de hamster.

À cet effet, des quantités variables de sérum contenant l'alpha $_1$ - protéinase inhibiteur et l'alpha $_2$ - macroglobuline, sont préincubées avec une dose connue d'élastase pancréatique de porc. Puis, après cette pré-icubation, on dose l'activité enzymatique résiduelle sur substrat synthétique, le STANA.

La réaction est arrêtée par addition d'acide acétique pur (100 µl).

- Mode opératoire.

On préincube à température ambiante pendant 30 minutes 80 ng d'enzyme (correspondant à une densité optique de 0,8 pour une heure d'incubation avec le substrat avec des quantités de sérum variant de 0 à 30 µl.

Le sérum de hamster est dilué au 1/20ème, ce qui permet d'obtenir une inhibition quantifiable mais pas trop importante, au bout d'une heure d'incubation avec l'élastase, après que l'enzyme et le sérum aient été mis en présence pendant 30 minutes, il y a formation d'un complexe enzyme-inhibiteur. On rajoute ensuite 20 µl de

substrat et du tampon en quantité suffisante pour 1 ml de solution finale.

On incube les préparations pendant une heure, puis on arrête la réaction en diminuant le pH par addition de 100 µl d'acide acétique pur.

L'activité élastasique résiduelle est déterminée par la densité optique à 410 nm. La courbe observée présente deux phases majeures.

- une phase de décroissance régulière de l'activité enzymatique en fonction de la quantité de sérum ajoutée,

- un plateau correspondant à un excès d'inhibiteurs sériques, laissant une partie de l'enzyme active.

En effet, l'alpha $_2$ macroglobuline n'inhibe que partiellement l'activité élastolytique, car elle laisse le centre actif de l'enzyme accessible aux petits substrats, tel celui utilisé, le STANA.

Lorsqu'on compare les capacités d'inhibition de protéases de sérums témoins et de sérums traités par les dérivés glycosaminoglycaniques, on se place dans la partie décroissante de la courbe pour avoir une comparaison valable et quantifiable des différents pouvoirs inhibiteurs sériques.

On rapporte ci-après les résultats obtenus avec l'héparine, le CY 216, le CY 222 et la fraction dp>12 sur différentes élastases, à savoir :

1) l'élastase leucocytaire de rat.

On mesure l'activité élastasique en % en fonction de la concentration en mg/ml un produit testé. Les résultats sont reproduits sur la figure 1 où l'on rapporte les courbes de ces variations, respectivement avec l'héparine courbe-O-O, le CY 216 courbe -X-X, le CY 222, courbe -.-.- et la fraction dp>112, courbe Δ-Δ.

L'examen de ces courbes montre que les produits testés inhibent environ 80 % de l'activité de l'élastase

à une concentration aussi faible que 0,05 mg/ml.

- Sur la cathepsine G.

On mesure l'activité enzymatique résiduelle en fonction du produit testé.

Sur la figure 2, on rapporte les résultats obtenus avec l'héparine (a), CY 216 (b), CY 222 (c), la fraction dp12 (d), la fraction dp>12 (e) pour l'élastase et pour la cathepsine G .

L'examen de ces courbes montre la bonne corrélation entre l'inhibition exercée par ces produits sur l'élastase leucocytaire et sur la cathepsine G.

On constate que l'héparine, le CY 216 et le CY 222 inhibent plus fortement la cathepsine G que l'élastase leucocytaire alors que la fraction dp12 inhibe davantage l'élastase que la cathepsine :

- Sur l'élastase pancréatique de porc on opère comme suit :

On coule dans une boîte de Petri un gel d'agarose contenant de l'élastine fibreuse et l'on dépose dans des petits godets des quantités connues d'élastase avec ou sans inhibiteurs. On observe ensuite le développement des plages de lyse.

On constate une forte inhibition de l'action de l'élastase pancréatique à une forte concentration de 10 mg/ml des composés testés.

3) Protéase de type élastase (cellules musculaires lisses d'aortes de porc)(sérine protéase) et fibroblastes de l'adventice d'aorte de porcs (métallo protease).

A des concentrations supérieures à 10 mg/ml, on obtient une inhibition de ces élastases avec les produits testés.

EXEMPLE 2 : Effet sur l'activité élastasique et inhibitrice du sérum de hamster.

Inhibition de l'activité élastasique du sérum en

fonction de la concentration en produits testés.

On effectue le dosage sur substrat synthétique, le Suc(Ala)3-pNA (succinyl $(alanyl)_3$ paranitroanilide) et les résultats sont exprimés en pourcentages d'activité par rapport à 100 % de l'activité en référence du sérum seul sans addition des produits.

Ces résultats mettent en évidence une inactivation de l'activité élastolytique sérique en présence des molécules CY 216 et CY 222 ; ils montrent que même une concentration en ces produits aussi faible que 0,2 ng/ml est capable d'inactiver 20 % de l'activité élastolytique contenue dans 1 ml de sérum. Il existe une relation dose-effet de type exponentiel, surtout en ce qui concerne le produit CY 216. Aucune inactivation par l'héparine n'a été mise en évidence aux mêmes doses.

<u>EXEMPLE 3</u> : Etude in vivo de l'action des médicaments de l'invention sur l'équilibre élastase/anti-élastase dans le sérum de hamster normal ou traité.

On rapporte les résultats des expériences réalisées sur les trois groupes de hamsters se répartissant comme suit :

- 1er groupe = 10 témoins,
- 2ème groupe = 10 hamsters traités avec les molécules CY 216 pendant 6 semaines,
- 3ème groupe = 10 hamsters traités avec les molécules CY 222 pendant 6 semaines.

Ce traitement comporte une injection quotidienne de 2 mg de molécule, cette dose est considérée comme thérapeutique, pour un hamster de 100 g pendant 6 semaines.

a) Mesure de l'activité élastasique sérique circulante.

Pendant le traitement, on effectue des prélèvements sanguins chaque semaine au niveau de la jugulaire pour suivre les variations d'activité élastasique

circulante.

Les prélèvements sont réalisés 1, 2, 4, 7 et 24 heures après injection sous-cutanée des produits. ainsi, la cinétique d'évolution de la réponse enzymatique suite à l'administration des produits peut être suivie. Comme chaque groupe d'animaux est composé de 10 hamsters, 600 µl de sang sont prélevés sur deux hamsters de chaque groupe, 1 heure après l'injection sous-cutanée des produits à tester, puis 600 µl sont prélevés successivement aux temps 2 heures, 4 heures, 7 heures et 24 heures après l'injection sur 2 autres hamsters. Les activités élastasiques des sérums obtenus sont rapportées sur l'histogramme de la figure 3 (le temps de prélèvement du sang, après l'injection des produits à tester est indiqué en bas de colonne ainsi que la durée totale du traitement avant l'expérience (1,2 et 3 semaines).

Les résultats montrent que, dès la première semaine de traitement, on peut mettre en évidence une diminution de l'activité élastasique ; cette diminution est maximale au bout de 2 heures après injection des molécules de PM inférieur à l'héparine. Puis, on observe une tendance à revenir aux valeurs normales (données par celles des sérums de hamsters non traités) au bout de 24 heures.

Cet effet est mis en évidence pendant les 6 semaines de traitement, de manière comparable au fur et à mesure que se déroule le traitement.

Ces résultats permettent de conclure que l'effet des substances CY vis-à-vis de l'activité élastolytique sérique des animaux traités s'exerce dans le sens d'une inactivation comme celui observé sur l'activité élastolytique du sérum "in vitro" dans lequel les molécules CY ont été directement rajoutées au sérum. De plus, ces résultats observés "in vivo" suggèrent que l'effet diminue en fonction de la concentration décroissante en CY

dans le sang circulant, ce qui confirme également les résultats obtenus in vitro.

b) Mesure de la capacité d'inhibition sérique vis-à-vis de l'élastase pancréatique.

Le protocole suivi est le même que celui décrit précédemment. Pendant les trois semaines de traitement, on note une augmentation du taux sérique des inhibiteurs d'élastases. Cette expérience montre l'intérêt thérapeutique de l'héparine, de ses fractions et fragments ainsi que de ses dérivés sursulfatés dans l'emphysème qui est caractérisé par une perte du tissu élastique pulmonaire attribuée à une augmentation de l'activité élastolytique souvent combinée avec une diminution de la capacité d'inhibition vis-à-vis de l'élastase.
Capacité d'inhibition (voir Bulletin Européen de Physiopathologie Respiratoire (1980) Vol. 16. Supplément, particulièrement pages 199 - 206, et autres articles de la même publication).

Après quatre semaines de traitement, on peut mettre en évidence une tendance à l'augmentation du pouvoir inhibiteur vis-à-vis de l'élastase pancréatique (voir histogramme de la figure 4).

EXEMPLE 4 : Etude de l'effet des GAGs sur l'inhibition de la biosynthèse des collagènes.

On utilise des cellules musculaires lisses d'aortes de porc, 4ème passage, à raison de 2,5 x $10^6$ cellules par boîte. On ajoute comme traceur de la proline tritiée (50 pCi/boîte). On incube 24 heures. On utilise les séries suivantes : témoin (4 boîtes), héparine, CY 216 et CY 222 chacun à une dose de 20 µg et de 100 µg.

Après l'incubation, on prélève la couche cellulaire, on effectue une décantation du milieu, une opération de lavage, on dialyse contre $H_2O$ puis contre l'acide acétique 0,5 M, puis on effectue une

pepsinisation pour solubiliser le collagène (100 µg/ml, 2 x $24^n$, 4°C).

La fraction pepsine soluble contient 95 % de l'hydroxyproline radioactive totale. On précipite à l'aide de $(NH_4)_2 SO_4$ (172 mg/ml), puis on détermine la radioactivité de l'hydroxy ($^3H$) proline et de la ($^3H$) proline selon la méthode de Roj kind.

On effectue une électrophorèse en SDS-PAGE (Laemmli à 7,5 %).

On découpe les zones correspondant aux chaînes alpha 1 et alpha 2 et on détermine la radioactivité de chaque chaîne.

Les résultats obtenus sont rapportés dans le tableau ci-après.

L'examen de ces résultats montre l'effet inhibiteur important des GAGs vis-à-vis de la biosynthèse du collagène. On note un effet fonction de la dose avec l'héparine et le CY 216, l'inhibition étant plus importante à une dose de 100 µg que de 20 µg. En revanche, on observe une inhibition puissante avec le CY 222 dès l'utilisation de 20 µg.

| | | % du témoin | % hydroxy (3H) proline/total de la radioactivité | % de collagène Type III synthétisé/ Type I + Type III |
|---|---|---|---|---|
| Témoin | 2400 | | 1,3 % | 47 % |
| Héparine 20 µg<br>Héparine 100 µg | | 67 %<br>18 % | 1,1 %<br>0,4 % | 7 %<br>10 % |
| CY 216 20 µg<br>100 µg | | 77 %<br>23 % | 1,3 %<br>0,4 % | 28 %<br>24 % |
| CY 222 20 µg<br>100 µg | | 0 à 1 %<br>0 à 1 % | 0 à 0,05 %<br>0 à 0,05 % | 44 % } faible<br>37 % } volume |

Les GAGs utilisés pour l'élaboration des médicaments selon l'invention exercent en outre un effet qualitatif, inhibant sélectivement le type III de collagène par rapport aux types I+II.

EXEMPLE 5 : Effet des GAGs sur la biosynthèse de la fibronectine par les cellules musculaires lisses.

On utilise des cellules musculaires lisses d'aorte de porc, 4ème passage, 2,5 x $10^6$ cellules par boîte. On ajoute à chaque boîte utilisée en triple ou en quadruple pour chaque expérience, soit rien (témoin), soit 20 µg/ml de CY 216 ou 222, et à d'autres boîtes 100 µg/ml, c'est-à-dire 300 µg par boîte de CY 216 et 222 ainsi d'ailleurs que d'héparine.

Le traceur utilisé est la méthionine $^{35}S$, 5 µCi/boîte. On utilise deux temps d'incubation : 3 heures et 6 heures à cause de la rapidité de la biosynthèse de la fibronectine.

Après l'incubation et décantation du milieu et lavage, on effectue une extraction avec le deoxycholate pour obtenir la fraction membranaire et intracellulaire et on détermine par immunoprécipitation la fraction de radioactivité incorporée spécifiquement dans la fibronectine. Le tableau ci-joint montre l'incorporation à 3 heures et 6 heures dans l'extrait deoxycholate de la méthionine $^{35}S$ et le pourcentage de radioactivité retrouvée dans la bande de la fibronectine isolée de l'immunoprécipité par électrophorèse sur gel d'acrylamide. On peut constater une inhibition de l'ordre de 50 % de la fraction de l'isotope incorporé dans la fibronectine en présence des héparinoïdes.

EXEMPLE 6 : Inhibition de la biosynthèse de la fibronectine par les GAGs.

2,5 x $10^6$ cellules musculaires lisses d'aorte de porc par boîte - 20 µg/ml et 100 µg/ml de CY 216, CY 222 et d'héparine, 3 h et 6 h d'incubation.

Dosage de la radioactivité dans l'extrait deoxycholate (fraction membranaire) et dans l'immunoprécipité de la fibronectine, après séparation de sa bande sur gel de polyacrylamide, découpage et comptage de la radioactivité.

| | % de radioactivité incorporée, retrouvée dans la fibronectine | | | |
|---|---|---|---|---|
| | 3 h | % inhib. | 6 h | % inhib. |
| Témoin<br>Radioactivité totale dans l'extrait dpm/$10^6$ cellules : 45.800 x 3,5 = 160 300,00 ± 5 600 (s.d.) | 70,6 | | 31,7 | |
| CY 216 20 µg/ml | 39,2 | 44 | 25,0 | 21 |
| CY 216 100 µg/ml | 38,4 | 46 | 24,1 | 24 |
| CY 222 20 µg/ml | 36,1 | 49 | 21,0 | 34 |
| CY 222 100 µg/ml | 37,3 | 47 | 20,2 | 36 |
| Héparine 20 µg/ml | 35,1 | 50 | 20,2 | 36 |
| Héparine 100 µg/ml | 33,7 | 52 | 14,8 | 47 |

Ces résultats font apparaître l'effet inhibiteur des GAGs à l'égard de la biosynthèse de la fibronectine dans les conditions de l'essai.

EXEMPLE 7 : Etude in vitro de l'activité de GAGs s.s. sur l'élastase leucocytaire et la cathepsine G. ("s.s." désigne les GAGs à profil de sulfatation modifié).

L'élastase leucocytaire est préparée à partir de polymorphonucléaires de rats obtenus à partir d'un exsudat pleural. Le substrat est constitué par le N-succinyl trialanine paranitroanilide et l'activité enzymatique est déerminée après incubation de l'enzyme avec le produit à tester 1 heure à température ambiante (20°C). On

ajoute ensuite le substrat, on incube 20 heures à 37°C et on effectue une lecture à 410 nm.

Pour la cathepsine G humaine obtenue à partir de leucocytes polymorphonucléaires humains, le dosage est effectué sur un substrat d'azocaséine. Dans ce cas également, l'enzyme est incubée avec le produit à tester pendant 1 heure à température ambiante. On ajoute ensuite l'azocaséine et on incube le mélange pendant 5 heures à 37°C. On précipite l'acide trichloracétique à 5 % (concentration finale), on centrifuge et on effectue une lecture à 366 nm.

Les produits testés sont énumérés dans le tableau ci-après avec leur activité inhibitrice exercée sur l'élastase et la cathepsine G.

Les produits à profil de sulfatation non modifié sont également mentionnés.

Les essais rapportés dans la partie B' ont été réalisés en présence d'une concentration en NaCl de 0,18 M.

On note une bonne préservation de l'activité inhibitrice pour les différents dérivés testés.

L'hypersulfatation renforce l'inhibition (élastase inhibée à 100 % par le CY 216 hypersulfaté, cathepsine G inhibée à 59 % par l'héparine hypersulfatée et à 65 % par le CY 216 hypersulfaté). Pour une taille moléculaire donnée, l'inhibition est plus forte pour les produits hypersulfatés. Ce phénomène est bien entendu le plus visible pour les plus petits poids moléculaires qui n'ont quasiment aucune activité inhibitrice et qui sont fortement inhibiteurs lorsqu'ils sont hypersulfatés.

| Produit testé | % inhibition | | | |
|---|---|---|---|---|
| | Elastase | | Cathepsine G | |
| | a. | b. | c. | d. |
| A) Héparine (sel de sodium) | 100 | 100 | 49 | 59 |
| B) Oligosaccharides obtenus par fractionnement de CY222 sur Sépharose : | | | | |
| dp : | | | | |
| 12 | 63 | | 50 | |
| 12 | 19 | | 45 | |
| 10 | 22 | | 30 | |
| B') dp : | | | | |
| 16 | 82 | 100 | 40 | 60 |
| 22 | 91 | | 53 | |
| 20 | 92 | | 54 | |
| 12 | 92 | 97 | 46 | 51 |
| 8 | 53 | 95 | 34 | 53 |
| 6 | 38 | 91 | 8 | 43 |
| 4 | 28 | 87 | 7 | 42 |
| C) CY 216, CY 216D et dérivés | | | | |
| - CY 216 (sel de sodium) | 90 | | 55 | |
| - CY 216D (sel de tétrabutyl ammonium) | 78 | 100 | 52 | |
| - CYD216 (O-acétylé) | 98 | | 49 | |

a et c correspondent aux mesures avec les produits ayant le taux de sulfatation de produits naturels ou de leurs fragments et b et d aux mesures avec les produits à profil de sulfatation modifié.

REVENDICATIONS

1. Utilisation pour l'obtention d'un médicament actif vis-à-vis des pathologies du tissu conjonctif d'un glycosaminoglycane (ou GAG) et/ou d'un fragment de GAG, le cas échéant, sous forme de sel pharmacologiquement acceptable.

2. Utilisation selon la revendication 1, caractérisée en ce que le GAG utilisé est à base des motifs alternés D-glucosamine-acide uronique (acide L-iduronique ou D-glucuronique) tels que rencontrés dans l'héparine ou l'héparane-sulfate.

3. Utilisation selon la revendication 2, caractérisée en ce que le GAG est de l'héparine ou de l'héparane-sulfate.

4. Utilisation selon la revendication 2, caractérisée en ce que le GAG possède un poids moléculaire inférieur à celui de l'héparine ou de l'héparane-sulfate, en particulier un poids moléculaire (PM) inférieur à 10 000 daltons environ.

5. Utilisation selon la revendication 4, caractérisée en ce que le GAG est choisi parmi :

- les mucopolysaccharides tels qu'obtenus par extraction alcoolique à partir d'héparine, formés d'un mélange de chaînes de poids moléculaires d'environ 2000 à 8000 et présentant des rapports des titres Yin-Wessler/USP d'au moins 2, en particulier les produits possédant un rapport YW/USP de l'ordre de 3 à 5 et des poids moléculaires moyens de 3000 à 5500 environ, désignés par l'expression CY 216 ;
- les mucopolysaccharides tels qu'obtenus par dépolymérisation ménagée de l'héparine à l'aide d'acide nitreux, ces produits présentant d'une manière générale les caractéristiques ci-dessus, mais étant terminés par des groupes de structure 2,5-anhydromanno, en particulier les mucopolysaccharides possédant une extrémité

2,5-anhydro-mannitol désignés par l'expression CY 222, ou encore une extrémité acide 2,5-anhydro-mannonique, ces produits étant formés de chaînes glycosidiques dont la majorité possède des PM de l'ordre de 2000 à 3000, notamment d'environ 2000 à 2600, et des rapports des titres Yin-Wessler/USP notamment d'au moins 10 avec des titres Yin-Wessler d'au moins 200 u/mg ;

- les mucopolysaccharides essentiellement formés de chaînes 1) ayant un poids moléculaire moyen de 3000 à 6000 daltons, notamment de 4000 à 5000 environ, 2) possédant un titre YW/USP inférieur à 10, notamment d'environ 6 à 3, 3) terminées par des motifs de structure 2,5-anhydromanno ;

- les oligosaccharides formés de 8 motifs sucre ou plus, de titre Yin-Wessler élevé pouvant atteindre 2000 u/mg et de titre USP plus faible, pratiquement nul, dotés d'une forte affinité pour l'AT-III, ces oligosaccharides pouvant être obtenus par dépolymérisation nitreuse ménagée et étant terminés par des groupes de structure 2,5-anhydromanno pouvant être obtenus en soumettant l'héparine à une dépolymérisation ménagée à l'aide d'une héparinase et comportant un motif de début de chaîne correspondant à un motif uronique insaturé, en particulier les octasaccharides de structure ABCDEFGH répondant à la formule ci-dessous :

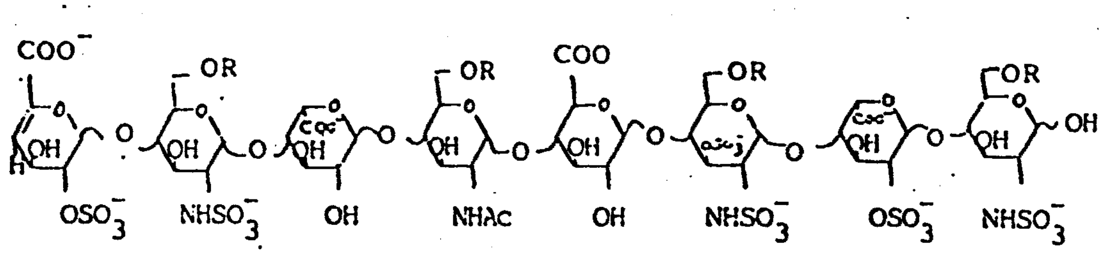

A B C D E F G H

- des compositions homogènes d'hexasaccharides telles qu'obtenues en soumettant les octasaccharides ci-dessus

à l'action ménagée d'une héparinase, en particulier les compositions répondant à la formule ci-dessous :

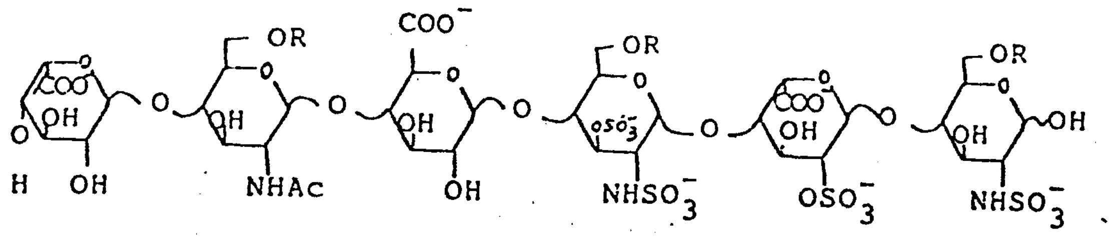

C' D E F G H

- des oligo- ou poly-saccharides de degré de polymérisation homogène, notamment, ceux obtenus par filtration, sur un matériau tel que du Sephadex, des mucopolysaccharides à activité antithrombotique tels qu'obtenus par extraction alcoolique, par exemple le CY 216, ou dépolymérisation nitreuse de l'héparine, par exemple le CY 222, ou par chromatographie par gradient de force ionique ;
- des GAGs tels qu'obtenus par action de periodate sur l'héparine, suivie de traitement en milieu basique ;
- des GAGs dits totaux, correspondant aux compositions de GAGs obtenues par traitement d'organes animaux ;
- des GAGs tels qu'obtenus par dépolymérisation de l'héparine avec de l'héparinase ou de l'acide nitreux, par séparation des fractions possédant la séquence de fixation à l'AT-III et gel filtration de ces fractions afin d'obtenir des fragments de GAGs de degré de polymérisation varié, par exemple d'une part des fragments de degré de polymérisation supérieur à 12 et d'autre part des fragments de GAGs de degré de polymérisation inférieur à 12, notamment de dp 12, 10, 8, 6 ou 4.

6. Utilisation selon la revendication 1, caractérisée en ce que le GAG mis en oeuvre est à base de motifs alternés D-galactosamine-acide uronique, tels que rencontrés par exemple dans le dermatane-sulfate, les

chondroïtines, les chondroïtines-sulfates ou encore l'acide hyaluronique, et en particulier est constitué par ces produits eux-mêmes.

7. Utilisation selon l'une quelconque des revendications précédentes, caractérisée en ce que le GAG mis en oeuvre est homogène au regard de son degré de polymérisation et notamment est formé de chaînes de degré de polymérisation pair.

8. Utilisation selon l'une quelconque des revendications précédentes, caractérisée en ce que le GAG mis en oeuvre possède un profil de sulfatation modifié, ce GAG étant constitué par exemple par l'un des produits définis ci-dessus dans lequel une partie, voire la majorité et même la totalité des groupes -OH sont remplacés par des groupes ester sulfurique $-OSO_3^-$.

9. Utilisation selon la revendication 8, caractérisée en ce que le degré de sulfatation du GAG varie de 2 à 4.

10. Utilisation selon l'une quelconque des revendications 1 à 9, caractérisée en ce que le GAG se présente sous forme d'une composition pharmaceutique appropriée pour l'administration par voie orale, injectable, rectale, de pommade, d'aérosols ou de liposomes.

11. Utilisation selon la revendication 10, caractérisée en ce que les solutions injectables renferment de 1 à 200 mg/ml de GAG, de préférence de 20 à 150 mg/ml lorsque ces solutions sont destinées à l'injection par voie sous-cutanée et de 30 à 100 mg/ml, notamment, de 40 à 50 mg/ml de GAG lorsqu'elles sont destinées à l'injection par voie intraveineuse ou par perfusion.

0208623

1/4

FIG.1

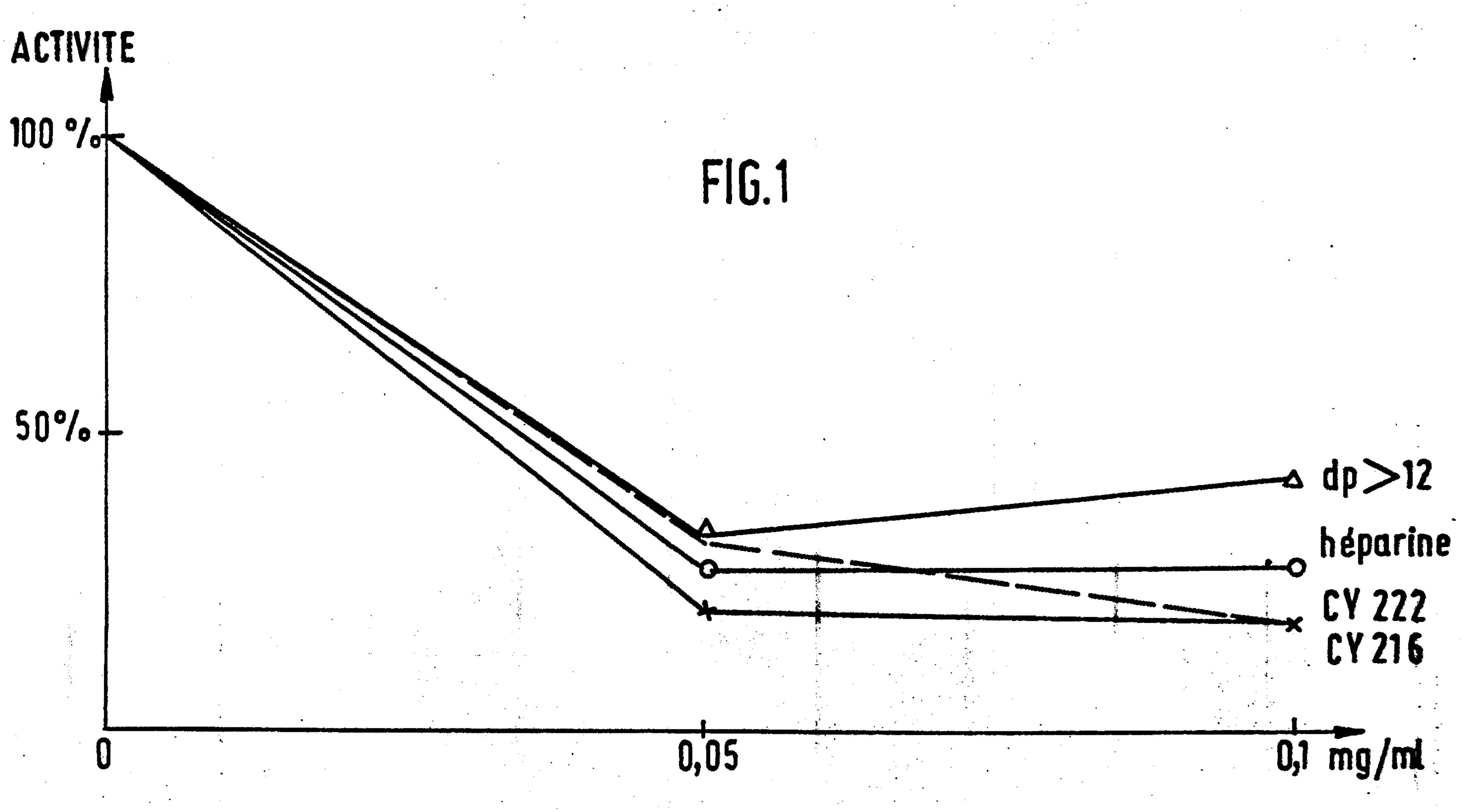

FIG. 2

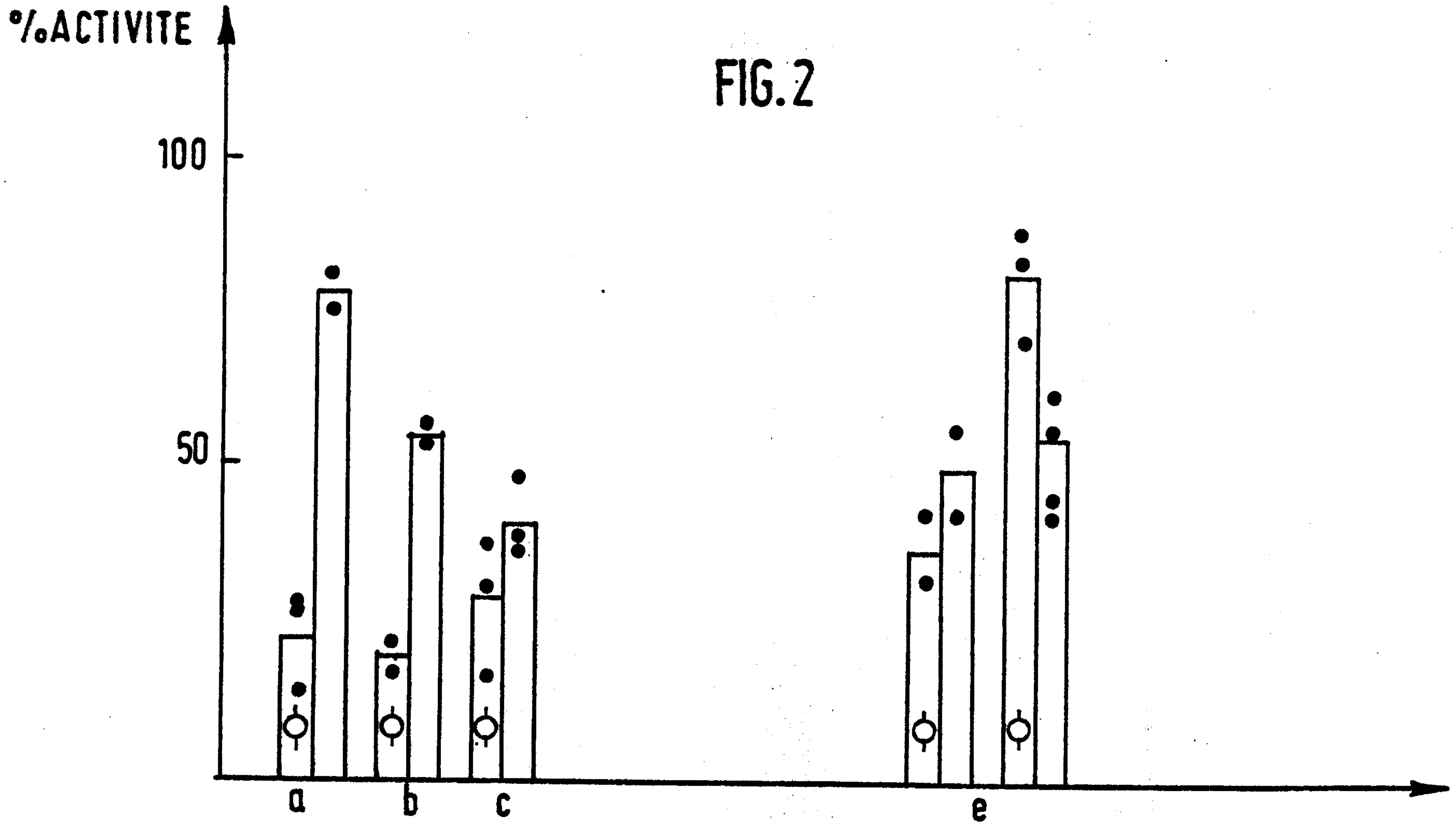

23.07.86





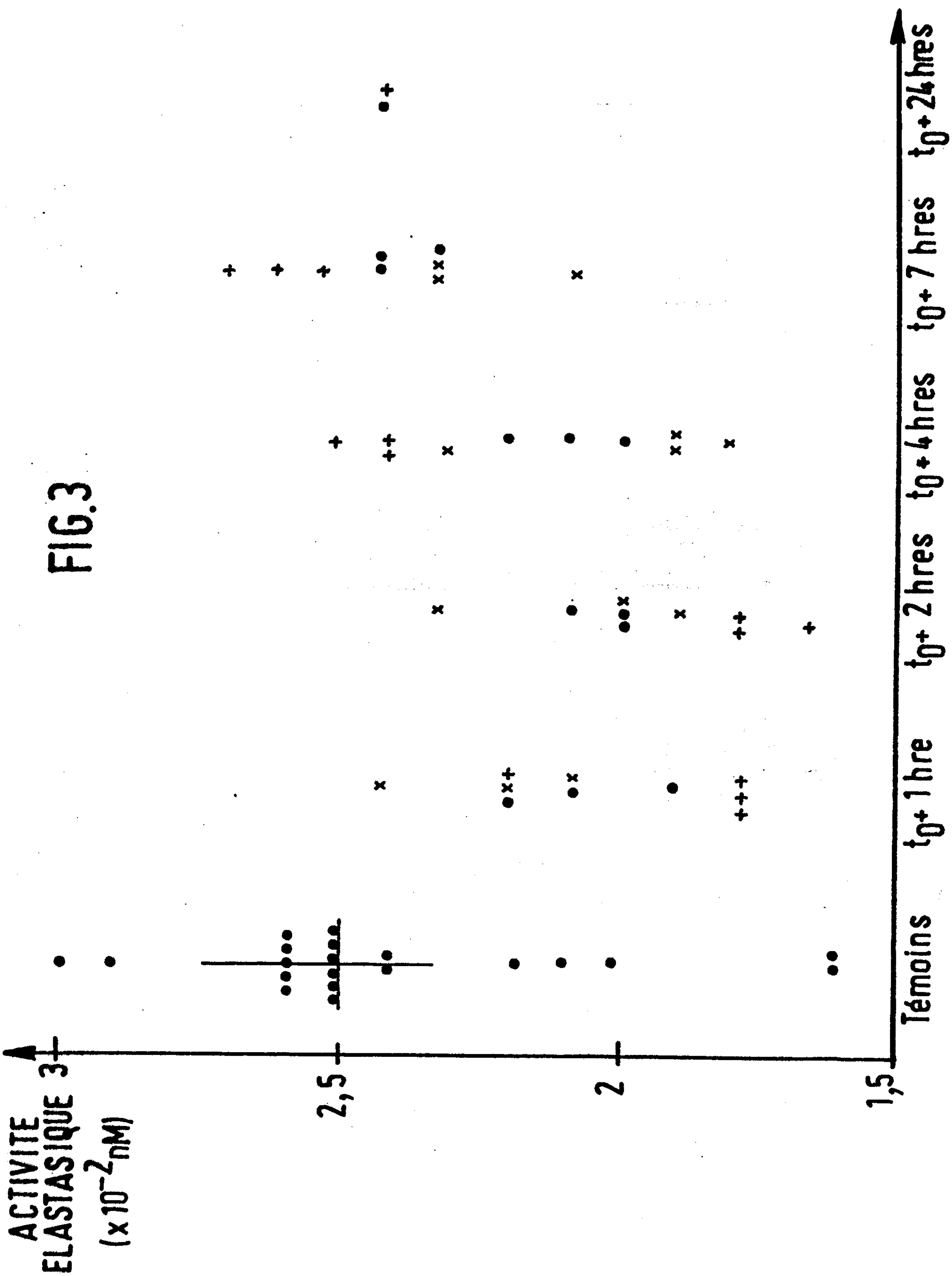
ACTIVITE ELASTASIQUE (x $10^{-2}$nM)
3
2,5
2
1,5
Témoins
$t_0$+ 1hre
$t_0$+ 2hres
$t_0$+ 4hres
$t_0$+ 7 hres
$t_0$+ 24hres

FIG.3

FIG. 4

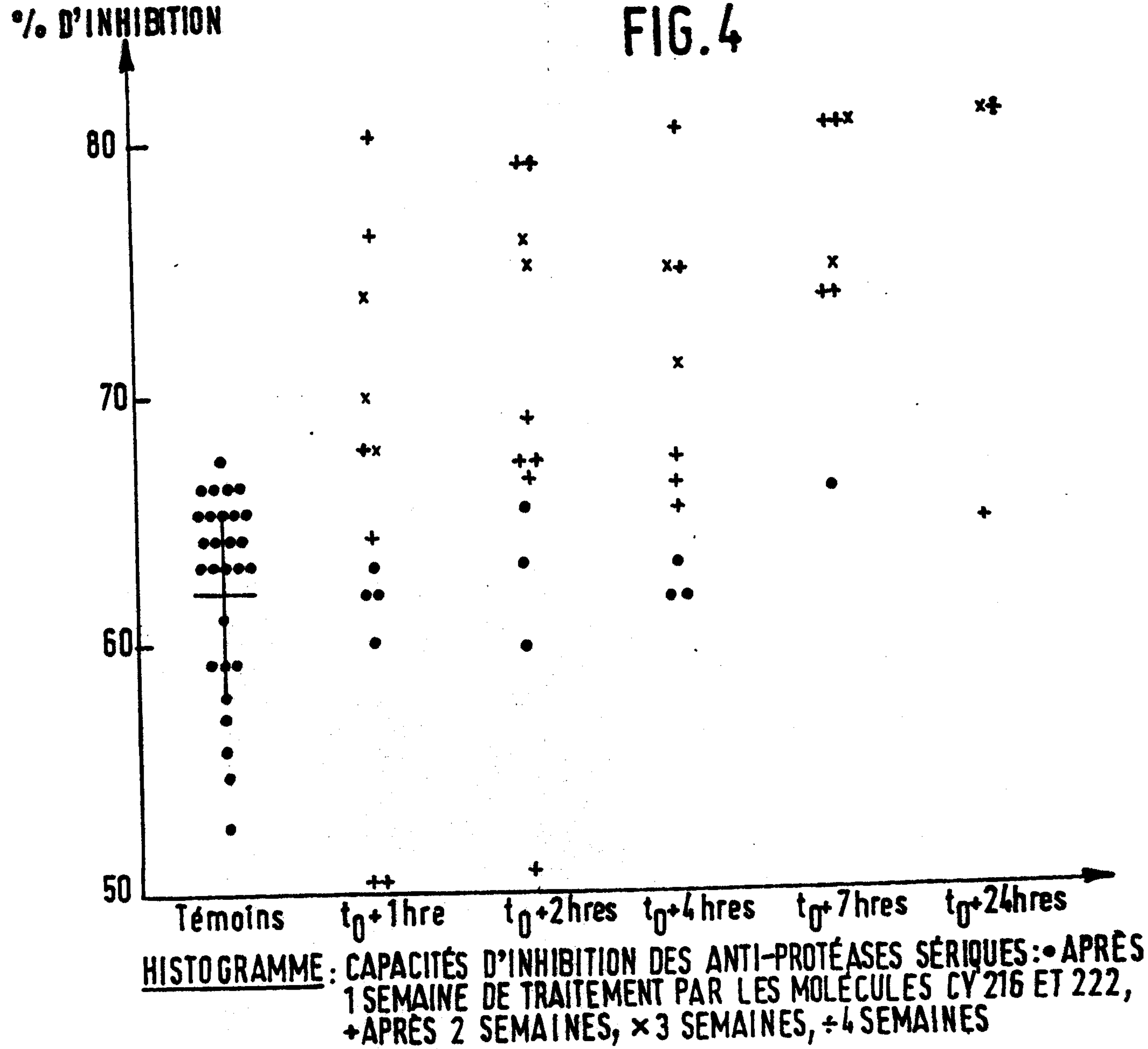


HISTOGRAMME: CAPACITÉS D'INHIBITION DES ANTI-PROTÉASES SÉRIQUES: • APRÈS 1 SEMAINE DE TRAITEMENT PAR LES MOLÉCULES CY 216 ET 222, + APRÈS 2 SEMAINES, × 3 SEMAINES, ÷ 4 SEMAINES